(19) Europäisches Patentamt
European Patent Office
Office européen des brevets

(11) Numéro de publication: **0 274 930 B1**

(12) # FASCICULE DE BREVET EUROPEEN

(45) Date de publication de fascicule du brevet: **21.08.91**

(51) Int. Cl.5: **C07D 471/04**, C07D 519/00, C07D 401/04, A61K 31/435, A61K 31/495, //(C07D471/04, 221:00,221:00),(C07D519/00, 487:00,471:00)

(21) Numéro de dépôt: **87402707.1**

(22) Date de dépôt: **01.12.87**

Le dossier contient des informations techniques présentées postérieurement au dépôt de la demande et ne figurant pas dans le présent fascicule.

(54) **Dérivés du pyrrole, leur préparation et les compositions pharmaceutiques qui les contiennent.**

(30) Priorité: **02.12.86 FR 8616795**

(43) Date de publication de la demande: **20.07.88 Bulletin 88/29**

(45) Mention de la délivrance du brevet: **21.08.91 Bulletin 91/34**

(84) Etats contractants désignés: **AT BE CH DE ES FR GB GR IT LI LU NL SE**

(56) Documents cités:
**EP-A- 0 174 858**
**EP-A- 0 271 404**
**EP-A- 0 274 929**
**US-A- 4 590 189**

**Chem. Soc. Rev. (1979) 8, 563-564**

(73) Titulaire: **RHONE-POULENC SANTE**
**20, avenue Raymond Aron**
**F-92160 Antony(FR)**

(72) Inventeur: **Bourzat, Jean-Dominique**
**35, rue Santos-Dumont**
**F-75015 Paris(FR)**
Inventeur: **Capet, Marc**
**10, rue de la Galaise**
**F-94320 Thiais(FR)**
Inventeur: **Cotrel, Claude**
**17 A, avenue du Dr Arnold Netter**
**F-75012 Paris(FR)**
Inventeur: **Labaudinière, Richard**
**11, rue du Château**
**F-94400 Vitry Sur Seine(FR)**
Inventeur: **Pitchen, Philippe**
**31, avenue de la Belle Image**
**F-94440 Marolles-En-Brie(FR)**

**EP 0 274 930 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

Inventeur: **Roussel, Gérard**
**20 ter, rue des Carrières**
**F-91450 Soisy Sur Seine(FR)**

(74) Mandataire: **Pilard, Jacques et al**
**RHONE-POULENC SANTE, Service Brevets,**
**20 Avenue Raymond Aron**
**F-92165 Antony Cédex(FR)**

**Description**

La présente invention concerne de nouveaux dérivés du pyrrole de formule générale :

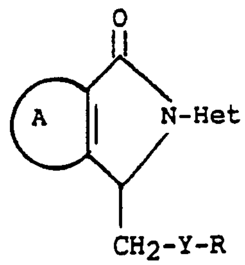

(I)

dans laquelle A forme avec le cycle pyrrole un noyau isoindoline, Het représente un radical naphtyridinyle ou quinolyle, non substitués ou substitués par un atome d'halogène ou un radical alcoyle (1 à 4 C), alcoyloxy (1 à 4 C), alcoylthio (1 à 4 C), Y représente un radical CO,C = NOH ou CHOH et R représente un radical alcényle contenant 3 à 10 atomes de carbone en chaîne droite ou ramifiée ou un radical alcoyle non substitué ou substitué par un radical alcoyloxy, cycloalcoyle contenant 3 à 6 atomes de carbone, dialcoylamino, phényle ou bien R représente un radioal pipéridyle-4 non substitué ou substitué par un radical alcoyle (1 à 4 C) étant entendu que les radicaux alcoyle sont en chaîne droite ou ramifiée et contiennent, sauf mention spéciale, 1 à 10 atomes de carbone, ainsi que, lorsqu'ils existent, leurs sels pharmaceutiquement acceptables et les isomères optiques des produits de formule (I).

Dans le brevet américain US 4 590 189 et la demande de brevet européen EP 174858 sont décrits des dérivés de l'isoindolinone substitués en position -3 par un radical carboxyméthyl ou ses dérivés qui sont actifs sur le système nerveux central et qui, en particulier, manifestent une activité anxiolytique.

Dans les demandes de brevets européens EP 271404 ET EP 274929 sont décrits des produits dont la structure est voisine de celle des produits de la présente demande.

Selon l'invention, les produits de formule générale (I) dans laquelle Y représente un radical CO et les autres symboles sont définis comme précédemment à l'exception pour Het de représenter un radical naphtyridine-1,8 yle-2 substitué par un atome d'halogène en position 7, peuvent être prépares par action d'une cétone de formule générale :

$CH_3$-CO-R (II)

dans laquelle R est défini comme précédemment sur une hydroxy-3 isoindolinone de formule générale :

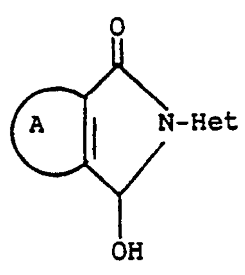

(III)

dans laquelle A et Het sont définis comme précédemment à l'exception pour Het de représenter un radical naphtyridine-1,8 yl-2 substitué en position 7 par un atome d'halogène.

On opère généralement dans un solvant aprotique dipolaire tel que le diméthylformamide ou la N-méthylpyrrolidone-2 en présence d'une base telle qu'un hydrure de métal alcalin comme l'hydrure de sodium à une température comprise entre -10 et +60 °C.

Les produits de formule générale (III) peuvent être préparés par application ou adaptation des méthodes décrites dans les brevets belges 793 851, 835 325 et 815 019.

Selon l'invention, les produits de formule générale (I) dans laquelle Y représente un radical CO et les autres symboles sont définis comme précédemment à l'exception pour Het de représenter un radical naphtyridine-1,8 yle-2 substitué en position 7 par un radical alcoyloxy ou alcoylthio peuvent être préparés par action d'un β-céto-ester de formule générale :

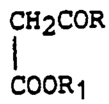

(IV)

dans laquelle $R_1$ représente un radical alcoyle et R est défini comme précédemment, sur un produit de formule générale :

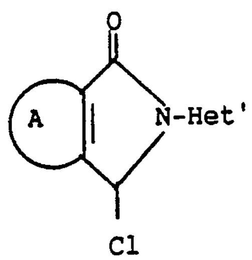

(V)

dans laquelle Het' a la définition donnée précédemment pour Het à l'exception de représenter un radical naphtyridine-1,8 yle-2 substitué en position 7 par un radical alcoyloxy ou alcoylthio et A est défini comme précédemment suivie d'une désalcoyloxycarbonylation de l'ester formé intermédiairement.

La condensation du produit de formule générale (IV) sur le produit de formule générale (V) s'effectue en général dans un solvant organique en présence d'une base, par exemple dans le diméthylformamide ou le tétrahydrofuranne en présence d'un hydrure alcalin tel que l'hydrure de sodium à une température comprise entre 0 et 60° C, de préférence entre 20 et 60° C.

La désalcoyloxycarbonylation ultérieure peut s'effectuer par toute méthode connue de l'homme du métier, notamment par saponification alcaline suivie d'une acidification et d'un chauffage à une température comprise entre 100 et 200° C, par hydrolyse acide et décarboxylation concomitante à une température comprise entre 100 et 200° C ou bien encore par chauffage dans le diméthylsulfoxyde en présence d'un halogénure de métal alcalin, par exemple le chlorure de lithium à une température comprise entre 150 et 180° C.

Les produits de formule générale (V) peuvent être préparés par chloruration d'un produit de formule générale (III).

On opère généralement en présence d'un agent de chloruration tel que le chlorure de sulfinyle ou l'oxychlorure de phosphore en présence de quantités catalytiques de diméthylformamide à une température comprise entre 20° C et la température de reflux du mélange réactionnel ou de tout autre agent connu de l'homme du métier permettant de transformer un radical hydroxy en radical chloro sans toucher au reste de la molécule.

Les produits de formule générale (I) préparés selon les deux procédés exposés précédemment peuvent être transformés en d'autres produits de formule générale (I) selon les méthodes habituelles. Ainsi :

a) Les produits de formule générale (I) dans laquelle Y représente un radical C=NOH et les autres symboles sont définis comme précédemment peuvent être obtenus par action de l,hydroxylamine sur un produit de formule générale (I) dans laquelle Y représente un radical CO et les autres symboles sont définis comme précédemment dans un solvant tel qu'un mélange hydroalcoolique, à une température comprise entre 20° C et le reflux du mélange réactionnel.

b) Les produits de formule générale (I) dans laquelle Het représente un radical naphtyridine-1,8 yle-2 substitué en position 7 par un atome de brome ou de chlore et les autres symboles sont définis comme précédemment peuvent être préparés par action d'un agent d'halogénation tel que l'oxybromure ou l'oxychlorure de phosphore sur un produit de formule générale (I) dans laquelle Het représente un radical naphtyridine-1,8 yle-2 substitué en position 7 par un radical alcoyloxy ou alcoylthio, et les autres symboles sont définis comme précédemment en opérant au reflux du mélange réactionnel.

c) les produits de formule générale (I) dans laquelle Y représente un radical CHOH et les autres symboles sont définis comme précédemment peuvent être préparés par réduction des produits correspondants dans lesquels Y représente un radical CO et les autres symboles sont définis comme précédemment.

La réduction s'effectue par tout moyen connu de l'homme du métier pour réduire une cétone en alcool

sans toucher au reste de la molécule, par exemple au moyen de tétrahydruroborate de potassium dans un mélange d'alcool et d'eau à une température voisine de 20° C.

Comme l'homme du métier pourra s'en rendre compte, certains radicaux entrant dans la définition du symbole R sont incompatibles avec des réactifs mis en oeuvre au cours des réactions et doivent être protégés préalablement à la mise en oeuvre des procédés ou de certaines phases des procédés exposés précédemment. C'est notamment le cas lorsque le radical R contient des fonctions amines primaires ou secondaires ou des fonctions hydroxylées susceptibles de donner naissance à des réactions secondaires en présence d'hydrures métalliques ou de réactifs d'halogénation. Dans ce cas, les dites fonctions devront être protégées par toute méthode connue de l'homme du métier puis être débloquées après réaction.

Les nouveaux produits de formule générale (I) peuvent être purifiés par les méthodes connues habituelles, par exemple par cristallisation, chromatographie ou extractions successives en milieu acide et basique.

Les nouveaux produits de formule générale (I) peuvent être transformés en sel d'addition avec les acides par action d'un acide dans l'eau ou un solvant organique tel qu'un alcool, une cétone, un éther ou un solvant chloré. Le sel formé précipite, éventuellement après concentration de sa solution ; il est séparé par filtration ou décantation.

Les produits de formule générale (I) présentent des propriétés pharmacologiques particulièrement intéressantes, révélatrices d'une activité anxiolytique, hypnotique, anticonvulsivante, antiépileptique et myorelaxante. C'est ainsi qu'ils présentent une bonne affinité in vitro pour les sites récepteurs à benzodiazépine à des concentrations dont les valeurs sont comprises entre 0,4 et 200 nM selon la technique décrite par J.C. BLANCHARD et L. JULOU, J. of Neurochemistry, 40, 601 (1983) inspirée des travaux de SQUIRES et BRAESTRUP, Nature, 266,732 (1977).

Chez l'animal (souris), ils se sont montrés actifs à des doses généralement comprises entre 0,5 et 200 mg/kg par voie orale vis-à-vis des convulsions induites par le pentétrazol selon une technique voisine de celle de EVERETT et RICHARDS, J. Pharmacol., 81, 402 (1944).

Les nouveaux produits de formule générale (I) et leurs sels présentent en outre une toxicité faible. Leur $DL_{50}$ est généralement supérieure à 300 mg/kg, par voie orale chez la souris.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoates, fumarates, maléates, méthanesulfonates, isoéthionates, théophylline-acétates, salicylates, phénolphtalinates, méthylène-bis-β-oxynaphtoates ou des dérivés de substitution de ces composés.

Les exemples suivants montrent comment l'invention peut être mise en pratique.

EXEMPLE 1

A une solution de 1,6 g d'hydroxy-3 (méthoxy-7 naphtyridine-1,8 yl-2)-2 isoindolinone-1 dans 20 cm3 de diméthylformamide anhydre, maintenue sous atmosphère d'argon, on ajoute, à une température voisine de -5° C, par petites portions, 0,5 g d'une suspension huileuse (50 % en poids) d'hydrure de sodium et agite la suspension obtenue pendant 30 minutes à une température voisine de -5° C. On ajoute alors une solution de 1,2 g de méthyl-5 hexanone-2 dans 5 cm3 de diméthylformamide anhydre et l'on poursuit l'agitation pendant 5 heures à une température voisine de 20° C. Le mélange réactionnel est versé ensuite dans 200 cm3 d'eau distillée et extrait par 3 fois 100 cm3 de dichlorométhane. Les phases organiques sont réunies, lavées par 5 fois 20 cm3 d'eau distillée puis concentrées à sec sous pression réduite (2,7 kPa) à 40° C. Le résidu huileux obtenu est purifié par chromatographie sur 15 g de gel de silice contenus dans une colonne de 1,5 cm de diamètre [éluant : dichlorométhane-méthanol (99-1 en volumes)]. On élue d'abord avec 30 cm3 de solvant : l'éluat correspondant est éliminé ; on élue ensuite avec 100 cm3 de solvant : l'éluat correspondant est concentré à sec sous pression réduite (2,7 kPa) à 40° C. Le résidu obtenu est recristallisé dans l'acétate d'éthyle; on obtient ainsi 1,3 g de (méthoxy-7 naphtyridine-1,8 yl-2)-2 (méthyl-5 oxo-2 hexyl)-3 isoindolinone-1 fondant à 150° C.

EXEMPLE 2

A une solution de 8,0 g d'hydroxy-3 (méthoxy-7 naphtyridine-1,8 yl-2)-2 isoindolinone-1 dans 125 cm3 de diméthylformamide anhydre maintenue sous atmosphère d'argon, on ajoute, à une température voisine de -5° C, par petites portions, 0,7 g d'hydrure de sodium. La suspension obtenue est agitée pendant 30

minutes à une température voisine de -5°C puis additionnée d'une solution de 3,5 g de diméthylamino-4 butanone-2 dans 5 cm3 de diméthylformamide anhydre. Le mélange est agité pendant 3 heures à une température voisine de 20°C puis est versé dans 500 cm3 d'eau distillée et extrait par 3 fois 200 cm3 de dichlorométhane. Les phases organiques sont réunies, lavées par 4 fois 25 cm3 d'eau distillée, séchées sur du sulfate de magnésium, filtrées puis concentrées à sec sous pression réduite (2,7 kpa) à 40°C. Le résidu huileux obtenu est dissous dans 400 cm3 d'acétate d'éthyle et la solution obtenue est extraite par 2 fois 100 cm3 d'une solution aqueuse d'acide chlorhydrique 1N. Les phases aqueuses sont réunies, lavées par 50 cm3 d'acétate d'éthyle, alcalinisées par une solution aqueuse de soude 10N jusqu'à un pH voisin de 11 et extraites par 2 fois 250 cm3 d'acétate d'éthyle. Les phases organiques sont réunies, lavées par 3 fois 30 cm3 d'eau distillée, séchées sur du sulfate de magnésium, filtrées puis concentrées à sec sous pression réduite (2,7 kPa) à 60°C. Après recristallisation dans l'acétonitrile, on obtient 2,6 g de (diméthylamino-4 oxo-2 butyl)-3 (méthoxy-7 naphtyridine-1,8 yl-2)-2 isoindolinone-1 fondant à 140°C.

La diméthylamino-4 butanone-2 peut être obtenue par la méthode décrite par MANNICH C., Arch. Pharm., (1917), 255, 261.

L'hydroxy-3 (méthoxy-7 naphtyridine-1,8 yl-2)-2 isoindolinone-1 peut être préparée selon la méthode décrite dans le brevet belge 815 019.

EXEMPLE 3

A une suspension de 3,0 g de (méthoxy-7 naphtyridine-1,8 yl-2)-2 (méthyl-5 oxo-2 hexyl)-3 isoindolinone-1 dans 50 cm3 d'éthanol, on ajoute, à une température voisine de 20°C, 0,41 g de tétrahydruroborate de potassium en solution dans 12 cm3 d'eau distillée et agite la suspension pendant 16 heures à une température voisine de 20°C. Le mélange réactionnel est ensuite versé dans un mélange de 300 cm3 d'eau distillée et de 150 cm3 de dichlorométhane refroidi à une température voisine de 0°C. La phase aqueuse est séparée par décantation et réextraite par 2 fois 150 cm3 de dichlorométhane. Les phases organiques sont réunies, lavées par 3 fois 25 cm3 d'eau distillée, séchées sur du sulfate de magnésium, filtrées puis concentrées à sec sous pression réduite (2,7 kPa) à 40°C. Le produit obtenu est recristallisé 2 fois dans l'oxyde de diisopropyle. On obtient ainsi 2,0 g d'(hydroxy-2 méthyl-5 hexyl)-3 (méthoxy-7 naphtyridine-1,8 yl-2)-2 isoindolinone-1 fondant à 110°C.

EXEMPLE 4

En opérant d'une manière analogue à celle décrite à l'exemple 1, mais à partir de 9,2 g d'hydroxy-3 (méthoxy-7 naphtyridine-1,8 yl-2)-2 isoindolinone-1, de 2,9 g d'une suspension huileuse (50% en poids) d'hydrure de sodium, et de 6 g de cyclopropyl-1 propanone-2, on obtient, après recristallisation dans l'acétonitrile, 3,9 g de (cyclopropyl-3 oxo-2 propyl)-3 (méthoxy-7 naphtyridine-1,8 yl-2)-2 isoindolinone-1 fondant à 165°C.

La cyclopropyl-1 propanone-2 peut être préparée par la méthode décrite dans le brevet japonais 79 73, 757 [Chem. Abstr. (1979), 91, 174895g].

EXEMPLE 5

En opérant d'une manière analogue à celle décrite à l'exemple 1, mais à partir de 13,3 g d'hydroxy-3 (méthylthio-7 naphtyridine-1,8 yl-2)-2 isoindolinone-1, de 4,1 g d'une suspension huileuse (50% en poids) d'hydrure de sodium, de 9,4 g de méthyl-5 hexanone-2, et en agitant le mélange réactionnel pendant 4 heures à une température voisine de -5°C, on obtient, après recristallisation dans l'acétate d'éthyle, 4,3 g de (méthyl-5 oxo-2 hexyl)-3 (méthylthio-7 naphtyridine-1,8 yl-2)-2 isoindolinone-1, fondant à 160°C.

L'hydroxy-3 (méthylthio-7 naphtyridine-1,8 yl-2)-2 isoindolinone-1 peut être préparée de la manière suivante : A une suspension de 16,6 g de (méthylthio-7 naphtyridine-1,8 yl-2)-2 isoindolinedione-1,3 dans un mélange de 250 cm3 de tétrahydrofuranne et de 25 cm3 d'eau, on ajoute, à une température voisine de 20°C, par petites portions, 2,8 g de tétrahydruroborate de potassium et agite la suspension obtenue pendant 4 heures à une température voisine de 20°C. Le mélange réactionnel est ensuite versé dans un mélange de 80 g de glace et 160 cm3 d'eau et neutralisé avec une solution aqueuse d'acide chlorhydrique 1N. Le produit insoluble est séparé par filtration, lavé par 5 fois 50 cm3 d'eau et séché à l'air. On obtient ainsi, 13 g d'hydroxy-3 (méthylthio-7 naphtyridine-1,8 yl-2)-2 isoindolinone-1 fondant à 210°C.

La (méthylthio-7 naphtyridine-1,8 yl-2)-2 isoindolinedione-1,3 peut être préparée de la manière suivante : A une suspension de 9,6 g d'amino-2 méthylthio-7 naphtyridine-1,8 dans 275 cm3 de Dowtherm A (marque déposée), on ajoute 7,5 g d'anhydride phtalique. Le mélange réactionnel est agité et chauffé à une

température de 155 °C pendant 3 heures, puis refroidi à une température voisine de 20 °C et additionné de 175 cm3 d'oxyde de diisopropyle et agité pendant 1 heure. Le produit insoluble est séparé par filtration, lavé par 3 fois 40 cm3 d'oxyde de diisopropyle et séché à l'air. On obtient ainsi 15,2 g de (méthylthio-7 naphtyridine-1,8 yl-2)-2 isoindolinedione-1,3 fondant à 235 °C.

L'amino-2 méthylthio-7 naphtyridine-1,8 peut être préparée comme décrit dans la demande de brevet européen publiée sous le numéro 172 083.

EXEMPLE 6

En opérant d'une manière analogue à celle décrite à l'exemple 1, mais à partir de 6,2 g de (chloro-7 quinolyl-2)-2 hydroxy-3 isoindolinone-1, de 2 g d'une suspension huileuse (50% en poids) d'hydrure de sodium, de 4,6 g de méthyl-5 hexanone-2, et en agitant le mélange réactionnel pendant 3 heures à une température voisine de -10 °C, on obtient après recristallisation dans l'éthanol, 1,4 g de (chloro-7 quinolyl-2)-2 (méthyl-5 oxo-2 hexyl)-3 isoindolinone-1, fondant à 127 °C.

La (chloro-7 quinolyl-2)-2 hydroxy-3 isoindolinone-1 peut être préparée par la méthode décrite dans le brevet belge 793 851.

EXEMPLE 7

En opérant d'une manière analogue à celle décrite à l'exemple 1, mais à partir de 6,2 g d'hydroxy-3 (méthoxy-7 naphtyridine-1,8 yl-2)-2 isoindolinone-1, de 2 g d'une suspension huileuse (50% en poids) d'hydrure de sodium et de 3,1 g de cyclohexyl-1 propanone-2, on obtient, après recristallisation dans l'acétonitrile, 5 g de (cyclohexyl-3 oxo-2 propyl)-3 (méthoxy-7 naphtyridine-1,8 yl-2)-2 isoindolinone-1 fondant à 139 °C.

La cyclohexyl-1 propanone-2 peut être préparée par la méthode décrite par GUERBET M., C. R. Acad. Sc. Paris, (1917), 164, 952.

EXEMPLE 8

En opérant d'une manière analogue à celle décrite à l'exemple 1, mais à partir de 3,11 g d'hydroxy-3 (méthoxy-7 naphtyridine-1,8 yl-2)-2 isoindolinone-1, de 1 g d'une suspension huileuse (50% en poids) d'hydrure de sodium, de 2,6 g de méthyl-6 heptanone-2 et en agitant le mélange réactionnel pendant 4 heures à une température voisine de -5 °C, on obtient, après recristallisation dans l'acétonitrile, 4,4 g de (méthoxy-7 naphtyridine-1,8 yl-2)-2 (méthyl-6 oxo-2 heptyl)-3 isoindolinone-1 fondant à 140 °C.

La méthyl-6 heptanone-2 peut être préparée par la méthode décrite par BRUNIE J. C., COLONGE J., C. R. Acad. Sc. Paris (1962), 255, 1621.

EXEMPLE 9

En opérant d'une manière analogue à celle décrite à l'exemple 1, mais à partir de 3,1 g d'hydroxy-3 (méthoxy-7 naphtyridine-1,8 yl-2)-2 isoindolinone-1, de 1 g d'une suspension huileuse (50% en poids) d'hydrure de sodium, de 2,4 g de méthyl-4 hexanone-2, et en agitant le mélange réactionnel pendant 20 heures à une température voisine de 0 °C, on obtient, après recristallisation dans l'acétonitrile, 2,2 g de (méthoxy-7 naphtyridine-1,8 yl-2)-2 (méthyl-4 oxo-2 hexyl)-3 isoindolinone-1 fondant à 120 °C.

La méthyl-4 hexanone-2 peut être préparée par la méthode décrite par JOHNSON J.R. , HAGER F. D., Org. Synth., Coll. Vol. I, 351.

EXEMPLE 10

En opérant d'une manière analogue à l'exemple 1, mais à partir de 6,2 g d'hydroxy-3 (méthoxy-7 naphtyridine-1,8 yl-2)-2 isoindolinone-1, de 1,92 g d'une suspension huileuse (50% en poids) d'hydrure de sodium, de 4,5 g de méthyl-3 hexanone-2, on obtient, après deux recristallisations successives dans l'oxyde d'isopropyle 2,1 g de (méthoxy-7 naphtyridine-1,8 yl-2)-2 (méthyl-3 oxo-2 hexyl)-3 isoindolinone-1 fondant à 86 °C.

La méthyl-3 hexanone-2 peut être préparée par la méthode décrite par JONES E. J., Ann. Chem. Pharm. (1884), 226, 287.

EXEMPLE 11

A une solution de 10 g d'hydroxy-3 (méthoxy-7 naphtyridine-1,8 yl-2)-2 isoindolinone-1 dans 195 cm3 de diméthylformamide anhydre maintenue sous atmosphère d'azote, on ajoute, à une température voisine de -5 ° C, par petites portions, 3,1 g d'une suspension huileuse (50% en poids) d'hydrure de sodium et agite la suspension obtenue pendant 30 minutes à une température voisine de -5 ° C. On ajoute alors une solution de 6,5 g d'hexanone-2 dans 5 cm3 de diméthylformamide anhydre et l'on poursuit l'agitation pendant 8 heures à une température voisine de 0 ° C puis pendant 16 heures à une température voisine de 20 ° C. Le mélange réactionnel est ensuite versé dans 1,5 litre d'eau. Le solide obtenu est séparé par filtration, lavé à l'eau et séché à l'air. Après recristallisation dans l'éthanol, on obtient 3,6 g de (méthoxy-7 naphtyridine-1,8 yl-2)-2 (oxo-2 hexyl)-3 isoindolinone-1 fondant à 161 ° C.

EXEMPLE 12

A une solution de 10 g d'hydroxy-3 (méthoxy-7 naphtyridine-1,8 yl-2)-2 isoindolinone-1 dans 180 cm3 de diméthylformamide anhydre maintenue sous atmosphère d'azote, on ajoute à une température voisine de -5 ° C, 3,1 g d'une suspension huileuse (50% en poids) d'hydrure de sodium et agite la suspension obtenue pendant 30 minutes à une température voisine de 0 ° C. On ajoute ensuite une solution de 8,2 g de cyclohexylméthylcétone dans 20 cm3 de diméthylformamide anhydre et l'on poursuit l'agitation pendant 6 heures à une température voisine de 0 ° C puis pendant 16 heures à une température voisine de 20 ° C. Le mélange réactionnel est ensuite versé dans 800 cm3 d'eau distillée. Le solide obtenu est séparé par filtration, lavé à l'eau, séché à l'air puis recristallisé dans l'acétonitrile et enfin purifié par chromatographie sous pression (50 kPa) sur 140 g de gel de silice (0,04 - 0,063 mm) contenus dans une colonne de 2,8 cm de diamètre. On élue par du chlorure de méthylène en recueillant des fractions de 20 cm3. Les fractions 25 à 62 sont réunies et concentrées à sec sous pression réduite (2,7 kpa) à 40 ° C. Le solide obtenu est repris avec de l'éther éthylique et est séparé par filtration. On obtient 4,8 g de (cyclohexyl-2 oxo-2 éthyl)-3 (méthoxy-7 naphtyridine-1,8 yl-2)-2 isoindolinone-1 fondant à 140 ° C.

EXEMPLE 13

En opérant comme à l'exemple 12, mais à partir de 15 g d'hydroxy-3 (méthoxy-7 naphtyridine-1,8 yl-2)-2 isoindolinone-1, 4,7 g d'une suspension huileuse (50% en poids) d'hydrure de sodium et 12,4 g de méthyl-6 heptène-5 one-2, on obtient après recristallisation dans l'éthanol, 7,6 g de (méthoxy-7 naphtyridine-1,8 yl-2)-2 méthyl-6 oxo-2 heptène-5 yl)-3 isoindolinone-1 fondant à 160 ° C.

EXEMPLE 14

En opérant comme à l'exemple 11, mais à partir de 10 g d'hydroxy-3 (méthoxy-7 naphtyridine-1,8 yl-2)-2 isodindolinone-1, 3,1 g d'une suspension huileuse (50% en poids) d'hydrure de sodium et 9,2 g d'acétyl-4 méthyl-1 pipéridine, on obtient, après recristallisation dans l'éthanol, 4,8 g de (méthoxy-7 naphtyridine-1,8 yl-2)-2 [(méthyl-1 pipéridyl-4)-2 oxo-2 éthyl]-3 isoindolinone-1 fondant à 172 ° C.

L'acétyl-4 méthyl-1 pipéridine peut être préparée de la façon suivante : A une solution de 5,2 g d'acétyl-4 pipéridine dans 10 cm3 d'eau distillée, on ajoute à une température voisine de 5 ° C, 3,9 cm3 d'acide formique puis 8,2 cm3 d'une solution aqueuse à 35% de formaldéhyde. Le mélange réactionnel est chauffé à reflux pendant 4 heures. On ajoute ensuite 50 cm3 d'une solution aqueuse de soude 1N et la solution aqueuse obtenue est extraite par 3 fois 60 cm3 de chlorure de méthylène. Les phases organiques sont réunies et lavées avec 2 fois 40 cm3 d'eau, séchées sur sulfate de magnésium et concentrées à sec sous pression réduite (2,7 kPa). On obtient ainsi 1,4 g d'acétyl-4 méthyl-1 pipéridine employée brute dans les synthèses ultérieures.

EXEMPLE 15

A une solution de 15 g d'hydroxy-3 (méthoxy-7 naphtyridine-1,8 yl-2)-2 isoindolinone-1 dans 280 cm3 de diméthylformamide anhydre maintenue sous atmosphère d'azote, on ajoute, à une température voisine de 0 ° C, 4,7 g d'une suspension huileuse (50% en poids) d'hydrure de sodium et agite la suspension obtenue pendant 30 minutes à une température voisine de 0 ° C. On ajoute une solution de 14,4 g de benzylacétone dans 20 cm3 de diméthylformamide anhydre et l'on poursuit l'agitation pendant 21 heures à une température voisine de 0 ° C. Le mélange réactionnel est ensuite versé sur 1,5 litre d'eau distillée. Le pH de la phase aqueuse est amené au voisinage de 5 par addition d'une solution aqueuse d'acide chlorhydrique 4N. On extrait avec 3 fois 500 cm3 de chlorure de méthylène. Les phases organiques sont

réunies et lavées avec 3 fois 80 cm3 d'eau distillée, séchées sur sulfate de magnésium et concentrées à sec à 40° C sous pression réduite (2,7 kPa). Le résidu huileux obtenu est purifié par chromatographie sous pression (50 kPa) sur 200 g de gel de silice (0,040 - 0,063 mm) contenus dans une colonne de 2,8 cm de diamètre. On élue avec du chlorure de méthylène en recueillant des fractions de 50 cm3. Les fractions 9 à 21 sont réunies et concentrées à sec à 40° C sous pression réduite (2,7 kPa). Après recristallisation dans l'éthanol du solide obtenu, on obtient 3,4 g de (méthoxy-7 naphtyridine-1,8 yl-2)-2 (oxo-2 phényl-4 butyl)-3 isoindolinone-1 fondant à 180° C.

EXEMPLE 16

A une suspension de 4,3 g de (méthoxy-7 naphtyridine-1,8 yl-2)-2 (méthyl-5 oxo-2 hexyl)-3 isoindolinone-1 dans 100 cm3 d'un mélange d'eau et d'éthanol (50-50 en volumes) maintenue à 0° C avec un bain d'eau glacée, on ajoute 0,9 g de chlorhydrate d'hydroxylamine. On laisse revenir la suspension obtenue jusqu'à une température voisine de 20° C et ajoute une solution de 0,7 g de carbonate de sodium dans 10 cm3 d'eau distillée. La suspension obtenue est chauffée au reflux pendant 22 heures. Au cours de cette période, on ajoute en deux fois 80 cm3 d'éthanol. On maintient le reflux pendant 12 heures en ajoutant en trois fois, 1,35 g de chlorhydrate d'hydroxylamine supplémentaires et 1,05 g de carbonate de sodium. Le mélange réactionnel est ensuite versé sur 1 litre d'eau. La phase aqueuse est extraite avec 3 fois 300 cm3 de chlorure de méthylène. Les phases organiques sont réunies, lavées avec 2 fois 100 cm3 d'eau distillée, séchées sur sulfate de magnésium, filtrées et concentrées à sec. Après recristallisation dans le propanol-2, on obtient 1 g de (hydroxyimino-2 méthyl-5 hexyl)-3 (méthoxy-7 naphtyridine-1,8 yl-2)-2 isoindolinone-1 fondant à 200° C.

La (méthoxy-7 naphtyridine-1,8 yl-2)-2 (méthyl-5 oxo-2 hexyl)-3 isoindolinone-1 peut être préparée comme il est décrit à l'exemple 1.

EXEMPLE 17

En opérant d'une manière analogue à celle décrite à l'exemple 1, mais à partir de 4,5 g d'hydroxy-3 (naphtyridine-1,8 yl-2)-2 isoindolinone-1, de 1,2 g d'une suspension huileuse (50% en poids) d'hydrure de sodium, de 3,7 g de méthyl-5 hexanone-2, et en agitant le mélange réactionnel pendant 3 heures à une température voisine de -10° C, on obtient, après recristallisation dans l'éthanol, 4,1 g de (méthyl-5 oxo-2 hexyl)-3 (naphtyridine-1,8 yl-2)-2 isoindolinone-1 fondant à 166° C.

L'hydroxy-3 (naphtyridine-1,8 yl-2)-2 isoindolinone-1 peut être préparée par la méthode décrite dans le brevet belge 815 019.

EXEMPLE 18

En opérant d'une manière analogue à celle décrite à l'exemple 1, mais à partir de 7,9 g d'hydroxy-3 (méthyl-7 naphtyridine-1,8 yl-2)-2 isoindolinone-1, de 2 g d'une suspension huileuse (50% en poids) d'hydrure de sodium, et de 6,2 g de méthyl-5 hexanone-2, et en agitant le mélange réactionnel pendant 3 heures à une température voisine de -5° C, puis pendant 2 heures à 0° C, on obtient, après recristallisation dans l'éthanol, 1 g de (méthyl-7 naphtyridine-1,8 yl-2)-2 (méthyl-5 oxo-2 hexyl)-3 isoindolinone-1 fondant à 155° C.

L'hydroxy-3 (méthyl-7 naphtyridine-1,8 yl-2)-2 isoindolinone-1 peut être préparée par la méthode décrite dans le brevet belge 815 019.

EXEMPLE 19

A une solution de 4,8 g de [(chloro-7 naphtyridine-1,8 yl-2)-2 oxo-3 isoindolinyl-1]-2 méthyl-6 oxo-3 heptanoate d'éthyle dans 50 cm3 de diméthylsulfoxide, maintenue sous atmosphère d'argon, on ajoute, à une température voisine de 20° C, 6,4 g de chlorure de lithium et 3,2 cm3 d'eau distillée. Le mélange réactionnel est chauffé à reflux pendant 30 minutes, puis refroidi à une température voisine de 40° C, additionné de 150 cm3 d'eau, et agité pendant 1 heure. Le solide qui précipite est séparé par filtration, lavé par 5 fois 10 cm3 d'eau, et séché à l'air. Après recristallisation dans l'acétonitrile, on obtient 3,3 g de (chloro-7 naphtyridine-1,8 yl-2)-2 (méthyl-5 oxo-2 hexyl)-3 isoindolinone-1 fondant à 180° C.

Le [(chloro-7 naphtyridine-1,8 yl-2)-2 oxo-3 isoindolinyl-1]-2 méthyl-6 oxo-3 heptanoate d'éthyle peut être préparé de la façon suivante : 1,73 g d'une suspension huileuse (50% en poids) d'hydrure de sodium sont ajoutés à 150 cm3 de diméthylformamide anhydre, sous atmosphpère d'argon, à une température

voisine de 0° C. On ajoute ensuite une solution de 8,37 g de méthyl-6 oxo-3 heptanoate d'éthyle dans 10 cm3 de diméthylformamide anhydre et la suspension obtenue est agitée pendant 35 minutes en laissant remonter la température vers 20° C. On ajoute enfin 10 g de chloro-3 (chloro-7 naphtyridine-1,8 yl-2)-2 isoindolinone-1 et maintient l'agitation pendant 6 heures à une température voisine de 20° C. Le mélange réactionnel est alors versé dans 800 cm3 d'eau. La phase aqueuse est acidifiée jusqu'à un pH voisin de 6 au moyen d'une solution aqueuse d'acide chlorhydrique 1N puis extraite par 3 fois 300 cm3 de dichlorométhane. Les phases organiques sont réunies, lavées par 3 fois 200 cm3 d'eau, séchées sur sulfate de magnésium puis concentrées à sec sous pression réduite (2,7 kPa). Après recristallisation du résidu obtenu dans 100 cm3 d'éthanol bouillant, on obtient 7 g de [(chloro-7 naphtyridine-1,8 yl-2)-2 oxo-3 isoindolinyl-1]-2 méthyl-6 oxo-3 heptanoate d'éthyle fondant à 140° C.

Le méthyl-6 oxo-3 heptanoate d' éthyle peut être préparé par la méthode décrite par KOGL F., SALEMINK C. A., Rec. Trav. Chim. Pays-Bas, (1952), 71, 779.

La chloro-3 (chloro-7 naphtyridine-1,8 yl-2)-2 isoindolinone-1 peut être préparée de la manière suivante : A 15,5 g d'hdyroxy-3 (méthoxy-7 naphtyridine-1,8 yl-2)-2 isoindolinone-1, on ajoute goutte à goutte sous agitation 200 cm3 de chlorure de sulfinyle. Le mélange réactionnel est chauffé à reflux sous agitation pendant 1 heure puis additionné de 0,5 cm3 de diméthylformamide et chauffé à nouveau à reflux pendant 3 heures, puis refroidi à une température voisine de 60° C et concentré à sec sous pression réduite (2,7 kPa) à 60° C. Au résidu obtenu, on ajoute 100 cm3 de dichlorométhane et concentre le mélange à sec sous pression réduite (2,7 kPa) à 60° C. Au solide résiduel obtenu on ajoute 100 cm3 de dichlorométhane et agite pendant 10 minutes. Le produit est séparé par filtration et lavé par 15 cm3 de dichlorométhane puis par 2 fois 25 cm3 d'oxyde de diisopropyle et séché à l'air. On obtient ainsi 12,4 g de chloro-3 (chloro-7 naphtyridine-1,8 yl-2)-2 isoindolinone-1, infusible à 300° C.

EXEMPLE 20

A une solution de 5 g de [(chloro-7 naphtyridine-1,8 yl-2)-2 oxo-3 isoindolinyl-1]-2 isopropoxy-4 oxo-3 butanoate d'éthyle dans 375 cm3 de diméthylsulfoxide anhydre, maintenue sous atmosphère d'argon, on ajoute, à une température voisine de 200° C, 7 g de chlorure de lithium et 3 cm3 d'eau distillée et chauffe le mélange réactionnel à reflux pendant 30 minutes. Après refroidissement à une température voisine de 40° C, on ajoute 800 cm3 d'eau glacée, et extrait ensuite par 3 fois 300 cm3 de dichlorométhane. Les phases organiques sont réunies, lavées par 3 fois 150 cm3 d'eau, séchées sur du sulfate de magnésium, filtrées puis concentrées à sec sous pression réduite (2,7 kPa) à 40° C. Le résidu huileux obtenu est purifié par chromatographie sur 300 g de silice contenus dans une colonne de 4 cm de diamètre [éluant : dichlorométhane - méthanol (98-2 en volumes)]. On élue d'abord avec 400 cm3 de solvant : l'éluat correspondant est éliminé; on élue ensuite avec 1800 cm3 de solvant : l'éluat correspondant est concentré à sec sous pression réduite (2,7 kPa) à 40° C. Après recristallisation du résidu obtenu dans l'acétonitrile, on obtient 2,5 g de (chloro-7 naphtyridine-1,8 yl-2)-2 (isopropoxy-3 oxo-2 propyl)-3 isoindolinone-1 fondant à 200° C.

Le [(chloro-7 naphtyridine-1,8 yl-2)-2 oxo-3 isoindolinyl-1]-2 isopropoxy-4 oxo-3 butanoate d'éthyle peut être préparé de la manière suivante : A une solution de 11 g d'isopropoxy-4 oxo-3 butanoate d'éthyle dans 150 cm3 de diméthylformamide anhydre, maintenue sous atmosphère d'argon et à une température voisine de 0° C, on ajoute, par petites portions, 2,15 g d'une suspension huileuse (50% en poids) d'hydrure de sodium, et agite la suspension obtenue pendant 30 minutes à une température voisine de 0° C. On ajoute alors une solution de 11,8 g de chloro-3 (chloro-7 naphtyridine-1,8 yl-2)-2 isoindolinone-1 dans 100 cm3 de diméthylformamide anhydre, et poursuit l'agitation pendant 3 heures à une température voisine de 20° C. Le mélange réactionnel est ensuite chauffé à une température de 60° C pendant 30 minutes puis refroidi à une température voisine de 10° C, versé dans 800 cm3 d'eau et additionné de 45 cm3 d'une solution aqueuse d'acide chlorhydrique 1N. Le produit qui précipite est séparé par filtration, lavé par 4 fois 100 cm3 d'eau distillée, et séché à l'air. Après deux recristallisations successives dans l'acétonitrile, on obtient 1,5 g de [(chloro-7 naphtyridine-1,8 yl-2)-2 oxo-3 isoindolinyl-1]-2 isopropoxy-4 oxo-3 butanoate d'éthyle fondant à 205° C.

L'isopropoxy-4 oxo-3 butanoate d'éthyle peut être préparé de la manière suivante : A 60 cm3 d'éther diéthylique anhydre, maintenue sous atmosphère d'argon, on ajoute, à une température voisine de 20° C, par petites portions, 25 g d'une suspension huileuse (50% en poids) d'hydrure de sodium puis 61,4 g de carbonate de diéthyle et chauffe le mélange réactionnel jusqu'à une température voisine de 45° C. On ajoute en 2 heures et 30 minutes, en maintenant cette température, 31 g d'isopropoxy-1 propanone-2. Après 1 heure d'agitation supplémentaire à cette température, le mélange réactionnel est refroidi à une température voisine de 10° C, et additionné successivement de 13 cm3 d'éthanol, 130 cm3 d'une solution

aqueuse d'acide chlorhydrique 4N et 400 cm3 d'éther diéthylique. La phase aqueuse est séparée par décantation et réextraite par 2 fois 200 cm3 d'éther diéthylique. Les phases organiques sont réunies, lavées successivement par 50 cm3 d'eau, 75 cm3 d'une solution aqueuse saturée de bicarbonate de sodium et, par 2 fois 50 cm3 d'eau, séchées sur du sulfate de magnésium, filtrées puis concentrées à sec sous pression réduite (2,7 kPa) à 40°C. Le résidu est purifié par distillation sous pression réduite. On obtient ainsi 50,5 g d'isopropoxy-4 oxo-3 butanoate d'éthyle sous forme d'un liquide incolore bouillant à 132-135°C sous 2,7 kPa.

L'isopropoxy-1 propanone-2 peut être préparée par la méthode décrite par HENZE H. R. et coll., J. Am. Chem. Soc., (1942), 64, 1223.

EXEMPLE 21

A une solution de 3 g de [(chloro-7 naphtyridine-1,8 yl-2)-2 oxo-3 isoindolinyl-1]-2 oxo-3 phényl-4 butanoate de méthyle dans 230 cm3 de diméthylsulfoxide, maintenue sous atmosphère d'argon, on ajoute, à une température voisine de 20°C, 4,2 g de chlorure de lithium et 1,8 cm3 d'eau et chauffe le mélange réactionnel à reflux pendant 45 minutes. Après refroidissement à une température voisine de 40°C, on ajoute 1500 cm3 d'eau glacée. Après 15 minutes d'agitation, le solide qui précipite est séparé par filtration, lavé par 5 fois 25 cm3 d'eau distillée, et séché à l'air. Le solide obtenu est purifié par chromatographie sur 140 g de gel de silice contenus dans une colonne de 3,4 cm de diamètre [éluant : dichlorométhane - méthanol (98-2 en volumes)]. On élue d'abord avec 200 cm3 de solvant : l'éluat correspondant est éliminé. On élue ensuite avec 900 cm3 de solvant : l'éluat correspondant est concentré à sec sous pression réduite (2,7 kPa) à 40°C. Après recristallisation dans l'acétonitrile, on obtient 1,2 g de (chloro-7 naphtyridine-1,8 yl-2)-2 (phényl-3 oxo-2 propyl)-3 isoindolinone-1 fondant à 182°C.

Le [(chloro-7 naphtyridine-1,8 yl-2)-2 oxo-3 isoindolinyl-1]-2 oxo-) phényl-4 butanoate de méthyle peut être préparé de la manière suivante : A une solution de 10,1 g d'oxo-3 phényl-4 butanoate de méthyle dans 130 cm3 de diméthylformamide anhydre, maintenue sous atmosphère d'argon, on ajoute, à une température voisine de -5°C, par petites portions, 2 g d'une suspension huileuse (50% en poids) d'hydrure de sodium, et agite la suspension obtenue pendant 30 minutes à 0°C. On ajoute alors une solution de 11,6 g de chloro-3 (chloro-7 naphtyridine-1,8 yl-2)-2 isoindolinone-1 dans 100 cm3 de diméthylformamide anhydre et poursuit l'agitation pendant 2 heures à une température voisine de 20°C. Le mélange réactionnel est ensuite agité pendant 30 minutes à une température voisine de 60°C puis versé après refroidissement dans 800 cm3 d'eau, acidifié jusqu'à un pH voisin de 2 au moyen d'une solution aqueuse d'acide chlorhydrique 5N, et extrait par 3 fois 300 cm3 de dichlorométhane. Les phases organiques sont réunies, lavées par 4 fois 150 cm3 d'eau, séchées sur du sulfate de magnésium, filtrées puis concentrées à sec sous pression réduite (2,7 kPa) à 40°C. Après recristallisation dans l'éthanol, on obtient 10,1 g de [(chloro-7 naphtyridine-1,8 yl-2)-2 oxo-3 isoindolinyl-1]-2 oxo-3 phényl-4 butanoate de méthyle fondant à 185°C.

L'oxo-3 phényl-4 butanoate de méthyle peut être préparé par la méthode décrite par HUNSDIECKER H., Chem. Ber., (1942), 75, 447.

EXEMPLE 22

En opérant d'une manière analogue à celle décrite à l'exemple 19, mais à partir de 6,1 g de [(chloro-7 naphtyridine-1,8 yl-2)-2 oxo-3 isoindolinyl-1]-2 méthyl-5 oxo-3 hexanoate d'éthyle, de 8,3 g de chlorure de lithium et de 4,2 cm3 d'eau distillée, on obtient, après recristallisation dans l'acétonitrile, 3,4 g de (chloro-7 naphtyridine-1,8 yl-2)-2 (méthyl-4 oxo-2 pentyl)-3 isoindolinone-1 fondant à 172°C.

Le [(chloro-7 naphtyridine-1,8 yl-2)-2 oxo-3 isoindolinyl-1]-2 méthyl-5 oxo-3 hexanoate d'éthyle peut être préparé en opérant d'une manière analogue à celle décrite à l'exemple 21, mais à partir de 2,6 g de méthyl-5 oxo-3 hexanoate d'éthyle, de 0,6 g d'une suspension huileuse (50% en poids) d'hydrure de sodium et de 3,3 g de chloro-3 (chloro-7 naphtyridine-1,8 yl-2)-2 isoindolinone-1. On obtient ainsi, après recristallisation dans l'oxyde de diisopropyle, 3,2 g de [(chloro-7 naphtyridine-1,8 yl-2)-2 oxo-3 isoindolinyl-1]-2 méthyl-5 oxo-3 hexanoate d'éthyle fondant à 145°C.

Le méthyl-5 oxo-3 hexanoate d'éthyle peut être préparé par la méthode décrite par KAGAN H.B., SUEN Y.H., Bull. Soc. Chim. France (1966), 6, 1819.

EXEMPLE 23

En opérant d'une manière analogue à celle décrite à l'exemple 19, mais à partir de 7,1 g de [(chloro-7 naphtyridine-1,8 yl-2)-2 oxo-3 isoindolinyl-1]-2 méthyl-4 oxo-3 pentanoate d'éthyle, de 10 g de chlorure de

lithium et de 5,1 cm3 d'eau distillée, on obtient, après recristallisation dans l'acétonitrile, 2,5 g de (chloro-7 naphtyridine-1,8 yl-2)-2 (méthyl-3 oxo-2 butyl)-3 isoindolinone-1 fondant à 212 °C.

Le [(chloro-7 naphtyridine-1,8 yl-2)-2 oxo-3 isoindolinyl-1]-2 méthyl-4 oxo-3 pentanoate d'éthyle peut être préparé en opérant d'une manière analogue à celle décrite à l'exemple 21, mais à partir de 4,7 g de méthyl-4 oxo-3 pentanoate d'éthyle, de 1,2 g d'une suspension huileuse (50% en poids) d'hydrure de sodium et de 6,6 g de chloro-3 (chloro-7 naphtyridine-1,8 yl-2)-2 isoindolinone-1. On obtient ainsi, après recristallisation dans l'oxyde de diisopropyle, 7 g de [(chloro-7 naphtyridine-1,8 yl-2)-2 oxo-3 isoindolinyl-1]-2 méthyl-4 oxo-3 pentanoate d'éthyle fondant à 146 °C.

Le méthyl-4 oxo-3 pentanoate d'éthyle peut être préparé par la méthode décrite par MOUREU C., DELANGE R., Bull. Soc. Chim. France, (1903), 29 (3), 666.

EXEMPLE 24

En opérant d'une manière analogue à celle décrite à l'exemple 19, mais à partir de 14,5 g de [(chloro-7 naphtyridine-1,8 yl-2)-2 oxo-3 isoindolinyl-1]-2 méthyl-7 oxo-3 octanoate d'éthyle, de 18,7 g de chlorure de lithium et de 9,5 cm3 d'eau distillée, on obtient, après recristallisation dans l'acétonitrile, 8,7 g de (chloro-7 naphtyridine-1,8 yl-2)-2 (méthyl-6 oxo-2 heptyl)-3 isoindolinone-1 fondant à 157 °C.

Le [(chloro-7 naphtyridine-1,8 yl-2)-2 oxo-3 isoindolinyl-1]-2 méthyl-7 oxo-3 octanoate d'éthyle peut être préparé en opérant d'une manière analogue à celle décrite à l'exemple 21, mais à partir de 12 g de méthyl-7 oxo-3 octanoate d'éthyle, de 2,3 g d'une suspension huileuse (50% en poids) d'hydrure de sodium et de 13,2 g de chloro-3 (chloro-7 naphtyridine-1,8 yl-2)-2 isoindolinone-1. On obtient ainsi, après recristallisation dans l'oxyde de diisopropyle, 11,6 g de [(chloro-7 naphtyridine-1,8 yl-2)-2 oxo-3 isoindolinyl-1]-2 méthyl-7 oxo-3 octanoate d'éthyle fondant à 135 °C.

Le méthyl-7 oxo-3 octanoate d'éthyle peut être préparé par la méthode décrite par MUKHERJI G., BARDHAN J. C., J. Chem. Soc., (1963), 2407.

EXEMPLE 25

En opérant d'une manière analogue à celle décrite à l'exemple 19, mais à partir de 13,7 g de [(chloro-7 naphtyridine-1,8 yl-2)-2 oxo-3 isoindolinyl-1]-2 méthyl-8 oxo-3 nonanoate d'éthyle, de 17,2 g de chlorure de lithium et de 8,8 cm3 d'eau, on obtient, après recristallisation dans l'acétonitrile, 8,7 g de (chloro-7 naphtyridine-1,8 yl-2)-2 (méthyl-7 oxo-2 octyl)-3 isoindolinone-1 fondant à 126 °C.

Le [(chloro-7 naphtyridine-1,8 yl-2)-2 oxo-3 isoindolinyl-1]-2 méthyl-8 oxo-3 nonanoate d'éthyle peut être préparé en opérant d'une manière analogue à celle décrite à l'exemple 21, mais à partir de 16,1 g de méthyl-8 oxo-3 nonanoate d'éthyle, de 2,9 g d'une suspension huileuse (50% en poids) d'hydrure de sodium et de 16,5 g de chloro-3 (chloro-7 naphtyridine-1,8 yl-2)-2 isoindolinone-1. On obtient ainsi, après recristallisation dans l'éthanol, 13,7 g de [(chloro-7 naphtyridine-1,8 yl-2)-2 oxo-3 isoindolinyl-1]-2 méthyl-8 oxo-3 nonanoate d'éthyle fondant à 136 °C.

Le méthyl-8 oxo-3 nonanoate d'éthyle peut être préparé de la manière suivante : A 250 cm3 d'éther diéthylique anhydre, maintenue sous atmosphère d'argon, on ajoute, à une température voisine de 0 °C, par petites portions, 48 g d'une suspension huileuse (50% en poids) d'hydrure de sodium puis 118 g de carbonate de diéthyle et chauffe le mélange réactionnel sous agitation, jusqu'à une température voisine de 45 °C. On ajoute ensuite en 2 heures et 30 minutes, en maintenant cette température, 71 g de méthyl-7 octanone-2. Après 1 heure d'agitation supplémentaire à 45 °C, le mélange réactionnel est refroidi à une température voisine de 10 °C, et additionné de 25 cm3 d'éthanol, puis d'une solution aqueuse d'acide chlorhydrique 4N jusqu'à un pH voisin de 6. La phase aqueuse est séparée par décantation et réextraite par 3 fois 100 cm3 d'éther diéthylique. Les phases organiques sont réunies, lavées par 2 fois 100 cm3 d'eau, séchées sur du sulfate de magnésium, filtrées puis concentrées à sec sous pression réduite (2,7 kPa) à 40 °C. Le résidu est purifié par distillation sous pression réduite. On obtient ainsi 78 g de méthyl-8 oxo-3 nonanoate d'éthyle sous forme d'un liquide incolore bouillant à 97 - 98 °C sous 0,13 kPa.

La méthyl-7 octanone-2 peut être préparée par la méthode décrite par HEILBRON I. M., JONES E. R. H., WEEDON B. C. L. , J. Chem. Soc., (1944), 140.

EXEMPLE 26

En opérant d'une manière analogue à celle décrite à l'exemple 19, mais à partir de 5,2 g de [(fluoro-7 naphtyridine-1,8 yl-2)-2 oxo-3 isoindolinyl-1]-2 méthyl-6 oxo-3 heptanoate d'éthyle, de 7,1 g de chlorure de lithium et de 3,6 cm3 d'eau distillée, on obtient, après recristallisation dans l'éthanol, 2,8 g de (fluoro-7

naphtyridine-1,8 yl-2)-2 (méthyl-5 oxo-2 hexyl)-3 isoindolinone-1 fondant à 162° C.

Le [(fluoro-7 naphtyridine-1,8 yl-2)-2 oxo-3 isoindolinyl-1]-2 méthyl-6 oxo-3 heptanoate d'éthyle peut être préparé en opérant d'une manière analogue à celle décrite à l'exemple 21, mais à partir de 3,8 g de méthyl-6 oxo-3 heptanoate d'éthyle, de 0,75 g d'une suspension huileuse (50% en poids) d'hydrure de sodium et de 4,2 g de chloro-3 (fluoro-7 naphtyridine-1,8 yl-2)-2 isoindolinone-1. On obtient ainsi, après recristallisation dans l'oxyde de diisopropyle, 4 g de [(fluoro-7 napthyridine-1,8 yl-2)-2 oxo-3 isoindolinyl-1]-2 méthyl-6 oxo-3 heptanoate d'éthyle fondant à 140° C.

Le méthyl-6 oxo-3 heptanoate d'éthyle peut être préparé selon la méthode décrite par KOGL F. et SALEMINK C.A., Rec. Trav. Chim. Pays-Bas (1952), 71, 779.

La chloro-3 (fluoro-7 naphtyridine-1,8 yl-2)-2 isoindolinone-1 peut être préparée en opérant d'une manière analogue à celle décrite à l'exemple 19, mais à partir de 6,6 g de (fluoro-7 naphtyridine-1,8 yl-2)-2 hydroxy-3 isoindolinone-1, de 40 cm3 de chlorure de sulfinyle et de 1,5 cm3 de diméthylformamide anhydre. On obtient ainsi 4,4 g de chloro-3 (fluoro-7 naphtyridine-1,8 yl-2)-2 isoindolinone-1, infusible à 265° C.

La (fluoro-7 naphtyridine-1,8 yl-2)-2 hydroxy-3 isoindolinone-1 peut être préparée de la manière suivante : A une suspension de 16,6 g de (fluoro-7 naphtyridine-1,8 yl-2)-2 isoindolinedione-1,3 dans un mélange de 90 cm3 de méthanol anhydre et de 90 cm3 de dioxanne, on ajoute, à une température voisine de 20° C, par petites portions, 2,3 g de tétrahydruroborate de potassium, et agite la suspension obtenue pendant 3 heures à une température voisine de 20° C. Le mélange réactionnel est ensuite versé dans un mélange de 120 g de glace et 240 cm3 d'eau. Le produit insoluble est séparé par filtration, lavé par 3 fois 50 cm3 d'eau, séché à l'air et recristallisé dans l'acétonitrile. On obtient ainsi 10,3 g de (fluoro-7 naphtyridine-1,8 yl-2)-2 hydroxy-3 isoindolinone-1 fondant à 246° C.

La (fluoro-7 naphtyridine-1,8 yl-2)-2 isoindolinedione-1,3 peut être préparée de la manière suivante : A une suspension de 20,6 g de (chloro-7 naphtyridine-1,8 yl-2)-2 isoindolinedione-1,3 dans 270 cm3 de nitrobenzène anhydre, maintenue sous atmosphère d'argon, on ajoute 15 g de fluorure de potassium et chauffe le mélange réactionnel à reflux sous agitation, pendant 22 heures. Après refroidissement à une température voisine de 80° C, le mélange réactionnel est concentré à sec sous pression réduite (0,13 kPa) à 80° C. Le résidu obtenu, est repris par 170 cm3 d'acétate d'éthyle. Le produit insoluble est séparé par filtration, lavé successivement par 30 cm3 d'acétate d'éthyle, 6 fois 30 cm3 d'eau et séché à l'air. On obtient ainsi 16,9 g de (fluoro-7 naphtyridine-1,8 yl-2)-2 isoindolinedione-1,3 fondant à 264° C.

La (chloro-7 naphtyridine-1,8 yl-2)-2 isoindolinedione-1,3 peut être préparée par la méthode décrite dans le brevet belge 835 325.

EXEMPLE 27

A une solution de 3 g de (méthoxy-7 naphtyridine-1,8 yl-2)-2 (méthyl-5 oxo-2 hexyl)-3 isoindolinone-1 dans 30 cm3 de dichlorométhane, maintenue sous atmosphère d'argon, on ajoute, à une température voisine de 20° C, une solution de 10,8 g de bromure de phosphoryle dans 30 cm3 de dichlorométhane, chauffe le mélange réactionnel à reflux pendant 5 heures, puis laisse agiter à une température voisine de 20° C pendant 16 heures. Le mélange est ensuite versé dans 50 g de glace, 100 cm3 d'eau et 200 cm3 de dichlorométhane, puis alcalinisé par une solution aqueuse saturée de carbonate de sodium jusqu'à un pH voisin de 10. La phase aqueuse est décantée et réextraite par 100 cm3 de dichlorométhane. Les phases organiques sont réunies, lavées par 6 fois 50 cm3 d'eau, séchées sur du sulfate de magnésium, filtrées puis concentrées à sec sous pression réduite (2,7 kPa). Après recristallisation dans l'acétonitrile, on obtient 2,4 g de (bromo-7 naphtyridine-1,8 yl-2)-2 (méthyl-5 oxo-2 hexyl)-3 isoindolinone-1 fondant à 166° C. La (méthoxy-7 naphtyridine-1,8 yl-2)-2 (méthyl-5 oxo-2 hexyl)-3 isoindolinone-1 peut être préparée comme décrit à l'exemple 1.

EXEMPLE 28

Une solution de 16,2 g de (méthoxy-7 naphtyridien-1,8 yl-2)-2 (méthyl-5 oxo-2 hexyl)-3 isoindolinone-1 dans 160 cm3 de chlorure de phosphoryle est chauffée à 100° C pendant 6 heures. Le mélange est ensuite refroidi à une température voisine de 50° C et concentré à sec sous pression réduite (2,7 kPa). Le résidu est additionné de 150 g de glace et 150 g d'eau. Le mélange est ensuite alcalinisé par une solution aqueuse d'ammoniac (d = 0,92) jusqu'à un pH voisin de 12, et extrait par 3 fois 300 cm3 d'acétate d'éthyle. Les phases organiques sont réunies, lavées par 4 fois 100 cm3 d'eau, séchées sur du sulfate de magnésium, filtrées puis concentrées à sec sous pression réduite (2,7 kPa). Le résidu obtenu est purifié par chromatographie sur 300 g de silice contenus dans une colonne de 4 cm de diamètre (éluant :

dichlorométhane). On élue d'abord avec 400 cm3 de solvant : l'éluat correspondant est éliminé; on élue ensuite avec 1800 cm3 de solvant et l'éluat correspondant est concentré à sec sous pression réduite (2,7 kPa). Après recristallisation du résidu obtenu dans l'acétonitrile, on obtient 6,4 g de (chloro-7 naphtyridine-1,8 yl-2)-2 (méthyl-5 oxo-2 hexyl)-3 isoindolinone-1 fondant à 180° C.

La (méthoxy-7 naphtyridine-1,8 yl-2)-2 (méthyl-5 oxo-2 hexyl)-3 isoindolinone-1 peut être préparée comme décrit à l'exemple 1.

La présente invention concerne également les médicaments qui contiennent les produits de formule (I) à l'état pur ou sous forme de compositions dans lesquelles ils sont associés à un adjuvant, un diluant et/ou un enrobage compatible et pharmaceutiquement acceptables. Ces médicaments peuvent être employés par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine pour leur action anxiolytique, hypnotique, anticonvulsivante, antiépileptique et myorelaxante.

En thérapeutique humaine, les doses dépendent de l'effet recherché et de la durée du traitement ; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

Les exemples suivants illustrent une composition selon l'invention.

EXEMPLE A

On prépare selon la technique habituelle des comprimés dosés à 10 mg de produit actif ayant la composition suivante :

- (méthoxy-7 naphtyridine-1,8 yl-2)-2 (méthyl-5 oxo-2 hexyl)-3 isoindolinone-1 .......................... 0,01 g
- amidon .................................................. 0,200 g
- silice précipitée .................................. 0,036 g
- stéarate de magnésium ........................... 0,004 g

En opérant de la même manière, on peut préparer des comprimés dont le principe actif est constitué des produits suivants :

- (chloro-7 naphtyridine-1,8 yl-2)-2 (méthyl-5 oxo-2 hexyl)-3 isoindolinone-1

- (méthoxy-7 naphtyridine-1,8 yl-2)-2 (oxo-2 hexyl)-3 isoindolinone-1
- (méthoxy-7 naphtyridine-1,8 yl-2)-2 (méthyl-5 oxo-2 hexyl)-3 isoindolinone-1
- (méthoxy-7 naphtyridine-1,8 yl-2)-2 (méthyl-6 oxo-2 heptèn-5 yl)-3 isoindolinone-1
- (méthoxy-7 naphtyridine-1,8 yl-2)-2 (méthyl-6 oxo-2 heptyl)-3 isoindolinone-1
- (cyclohexyl-3 oxo-2 propyl)-3 (méthoxy-7 naphtyridine-1,8 yl-2)-2 isoindolinone-1
- (chloro-7 naphtyridine-1,8 yl-2)-2 (isopropoxy-3 oxo-2 propyl)-3 isoindolinone-1
- (chloro-7 quinolyl-2)-2 (méthyl-5 oxo-2 hexyl)-3 isoindolinone-1
- (fluoro-7 naphtyridine-1,8 yl-2)-2 (méthyl-5 oxo-2 hexyl)-3 isoindolinone-1.

## Revendications

**Revendications pour les Etats contractants suivants : AT, BE, CH, DE, FR, GB, IT, LI, LU, NL, SE**

**1.** Nouveau dérivé du pyrrole, caractérisés en ce qu'ils répondent à la formule générale :

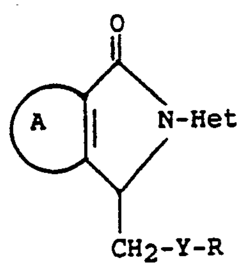

(I)

dans laquelle A forme avec le cycle pyrrole un noyau isoindoline, Het représente un radical naphtyridinyle ou quinolyle non substitués ou substitués par un atome d'halogène ou un radical alcoyle (1 à 4 C), alcoyloxy (1 à 4 C), alcoylthio (1 à 4 C), Y représente un radical CO,C=NOH ou CHOH et R représente un radical alcényle contenant 3 à 10 atomes de carbone en chaîne droite ou ramifiée ou un radical alcoyle non substitué ou substitué par un radical alcoyloxy, cycloalcoyle contenant 3 à 6 atomes de carbone, dialcoylamino, phényle, ou bien R représente un radical pipéridyle-4 non substitué ou substitué par un radical alcoyle (1 à 4 C), étant entendu que les radicaux alcoyle sont en chaîne droite ou ramifiée et contiennent, sauf mention spéciale, 1 à 10 atomes de carbone, ainsi que, lorsqu'ils existent, leurs sels pharmaceutiquement acceptables et les isomères optiques des produits de formule (I).

**2.** Procédé de préparation d'un produit selon la revendication 1 dans la formule duquel Y représente un radical CO et les autres symboles sont définis comme à la revendication 1 à l'exception pour Het de représenter un radical naphtyridine-1,8 yle-2 substitué par un atome d'halogène en position 7, caractérisé en ce que l'on fait agir un produit de formule générale :

$CH_3$-CO-R (II)

dans laquelle R est défini comme à la revendication 1, sur une hydroxy-3 isoindolinone de formule générale :

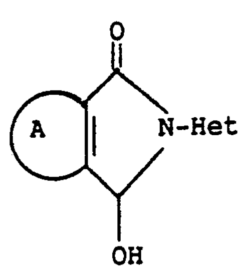

(III)

dans laquelle A et Het sont définis comme à la revendication 1 à l'exception pour Het de représenter un radical naphtyridine-1,8 yle-2 substitué en position 7 par un atome d'halogène, puis isole le produit

obtenu et le transforme éventuellement selon les méthodes connues en un autre produit de formule générale (I) et, si on le désire, le cas échéant, en un sel pharmaceutiquement acceptable.

**3.** Procédé de préparation d'un produit selon la revendication 1 dans la formule duquel Y représente un radical CO et les autres symboles sont définis comme à la revendication 1 à l'exception pour Het de représenter un radical naphtyridine-1,8 yle-2 substitué en position 7 par un radical alcoyloxy ou alcoylthio, caractérisé en ce que l'on fait agir un $\beta$-céto-ester de formule générale :

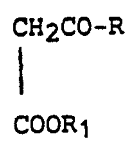

(IV)

dans laquelle R est défini comme à la revendication 1 et $R_1$ représente un radical alcoyle, sur un produit de formule générale :

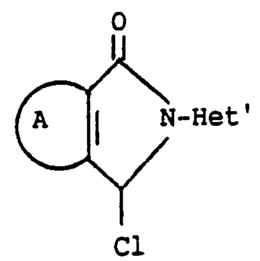

(V)

dans laquelle Het' a la définition donnée pour Het à la revendication 1 à l'exception de représenter un radical naphtyridine-1,8 yl-2 substitué en position 7 par un radical alcoyloxy ou alcoylthio et A est défini comme à la revendication 1, puis procède à une désalcoyloxycarbonylation de l'ester formé intermédiairement, isole le produit obtenu et le transforme éventuellement selon les méthodes connues en un autre produit de formule générale (I) et, si on le désire, le cas échéant, en un sel pharmaceutiquement acceptable.

**4.** Compositions pharmaceutiques, caractérisées en ce qu'elles contiennent au moins un produit selon la revendication 1, en association avec un ou plusieurs diluants ou adjuvants compatibles et pharmaceutiquement acceptables.

**Revendication pour les Etats contractants suivants : ES, GR**

**1.** Procédé de préparation de nouveaux dérivés de formule générale :

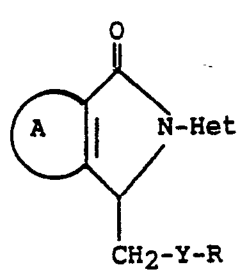

(I)

dans laquelle A forme avec le cycle pyrrole un noyau isoindoline, Het représente un radical naphtyridinyle ou quinolyle non substitués ou substitués par un atome d'halogène ou un radical alcoyle (1 à 4 C), alcoyloxy (1 à 4 C), alcoylthio (1 à 4 C), Y représente un radical CO,C = NOH ou CHOH et R représente

un radical alcényle contenant 3 à 10 atomes de carbone en chaîne droite ou ramifiée ou un radical alcoyle non substitué ou substitué par un radical alcoyloxy, cycloalcoyle contenant 3 à 6 atomes de carbone, dialcoylamino, phényle, ou bien R représente un radical pipéridyle-4 non substitué ou substitué par un radical alcoyle (1 à 4 C), étant entendu que les radicaux alcoyle sont en chaîne droite ou ramifiée et contiennent, sauf mention spéciale, 1 à 10 atomes de carbone, ainsi que, lorsqu'ils existent, leurs sels pharmaceutiquement acceptables et les isomères optiques des produits de formule (I), caractérisé en ce que :

A - Pour la préparation d'un produit de formule (I) dans laquelle Y représente un radical CO et les autres symboles sont définis comme précédemment à l'exception pour Het de représenter un radical naphtyridine-1,8 yle-2 substitué par un atome d'halogène en position 7, on fait agir un produit de formule générale :

$CH_3$-CO-R (II)

dans laquelle R est défini comme précédemment, sur une hydroxy-3 isoindolinone de formule générale :

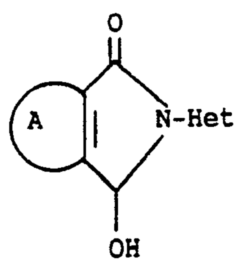

(III)

dans laquelle A et Het sont définis comme précédemment à l'exception pour Het de représenter un radical naphtyridine-1,8 yle-2 substitué en position 7 par un atome d'halogène, puis isole le produit obtenu et le transforme éventuellement selon les méthodes connues en un autre produit de formule générale (I) et, si on le désire, le cas échéant, en un sel pharmaceutiquement acceptable, ou en ce que :

B - Pour la préparation d'un produit de formule (I) dans laquelle Y représente un radical CO et les autres symboles sont définis comme précédemment à l'exception pour Het de représenter un radical naphtyridine-1,8 yle-2 substitué en position 7 par un radical alcoyloxy ou alcoylthio, on fait agir un β-céto-ester de formule générale :

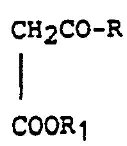

(IV)

dans laquelle R est défini comme précédemment et $R_1$ représente un radical alcoyle, sur un produit de formule générale :

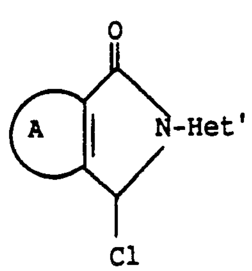

(V)

dans laquelle Het' a la définition donnée pour Het précédemment à l'exception de représenter un radical naphtyridine-1,8 yle-2 substitué en position 7 par un radical alcoyloxy ou alcoylthio et A est défini comme précédemment, puis procède à une désalcoyloxycarbonylation de l'ester formé intermédiairement, isole le produit obtenu et le transforme éventuellement selon les méthodes connues en un autre produit de formule générale (I) et, si on le désire, le cas échéant, en un sel pharmaceutiquement acceptable.

**Claims**

**Claims for the following Contracting States : AT, BE, CH, DE, FR, GB, IT, LI, LU, NL, SE**

1. A new pyrrole derivative, which is of the general formula:

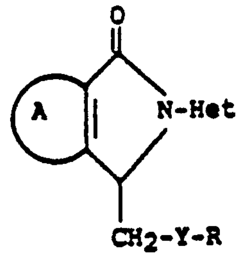

(I)

in which A forms with the pyrrole ring an isoindoline ring-system, Het denotes a naphthyridinyl or quinolyl radical, which is unsubstituted or substituted with a halogen atom or a (1 to 4 C) alkyl, (1 to 4 C) alkyloxy or (1 to 4 C) alkylthio radical, Y denotes a CO, C=NOH or CHOH radical and R denotes a straight- or branched-chain alkenyl radical containing 3 to 10 carbon atoms or an alkyl radical which is unsubstituted or substituted with an alkyloxy radical, with a cycloalkyl radical containing 3 to 6 carbon atoms, or with a dialkylamino or phenyl radical, or alternatively R denotes a 4-piperidyl radical which is unsubstituted or substituted with a (1 to 4 C) alkyl radical, on the understanding that the alkyl radicals are straight- or branched-chain radicals and contain, except where specifically stated, 1 to 10 carbon atoms, as well as, where they exist, their pharmaceutically acceptable salts and the optical isomers of the products of formula (I).

2. A process for preparing a product according to claim 1 in the formula of which Y denotes a CO radical and the other symbols are defined as in claim 1, with the exception of the option in which Het denotes a 1,8-naphthyridin-2-yl radical substituted with a halogen atom at the 7-position, wherein a product of general formula:

$CH_3$-CO-R (II)

in which R is defined as in claim 1, is reacted with a 3-hydroxyisoindolinone of general formula:

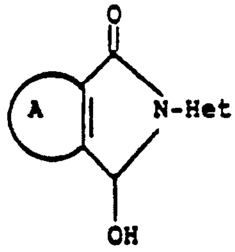

(III)

in which A and Het are defined as in claim 1, with the exception of the option in which Het denotes a 1,8-naphthyridin-2-yl radical substituted at the 7-position with a halogen atom, and the product obtained is then isolated and optionally converted according to known methods to another product of general formula (I) and, if so desired, where appropriate, to a pharmaceutically acceptable salt.

3. A process for preparing a product according to claim 1 in the formula of which Y denotes a CO radical and the other symbols are defined as in claim 1, with the exception of the option in which Het denotes a 1,8-naphthyridin-2-yl radical substituted at the 7-position with an alkyloxy or alkylthio radical, wherein a $\beta$-keto ester of general formula:

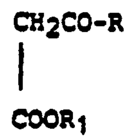

(IV)

in which R is defined as in claim 1 and $R_1$ denotes an alkyl radical, is reacted with a product of general formula:

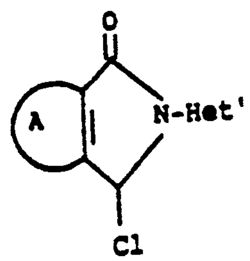

(V)

in which Het' has the definition given for Het in claim 1 with the exception of denoting a 1,8-naphthyridin-2-yl radical substituted at the 7-position with an alkyloxy or alkylthio radical, and A is defined as in claim 1, a dealkyloxycarbonylation of the ester formed as an intermediate is then carried out, and the product obtained is isolated and optionally converted according to known methods to another product of general formula (I) and, if so desired where appropriate, to a pharmaceutically acceptable salt.

4. Pharmaceutical compositions, which contain at least one product according to claim 1, in combination with one or more diluents or adjuvants which are compatible and pharmaceutically acceptable.

**Claim for the following Contracting States : ES, GR**

1. A process for preparing a new derivative of general formula:

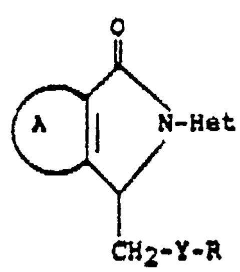

(I)

in which A forms with the pyrrole ring an isoindoline ring-system, Het denotes a naphthyridinyl or quinolyl radical, which is unsubstituted or substituted with a halogen atom or a (1 to 4 C) alkyl, (1 to 4 C) alkyloxy or (1 to 4 C) alkylthio radical, Y denotes a CO, C=NOH or CHOH radical and R denotes a straight- or branched-chain alkenyl radical containing 3 to 10 carbon atoms or an alkyl radical which is unsubstituted or substituted with an alkyloxy radical, with a cycloalkyl radical containing 3 to 6 carbon atoms, or with a dialkylamino or phenyl radical, or alternatively R denotes a 4-piperidyl radical which is unsubstituted or substituted with a (1 to 4 C) alkyl radical, on the understanding that the alkyl radicals are straight- or branched-chain radicals and contain, except where specifically stated, 1 to 10 carbon

atoms, as well as, where they exist, their pharmaceutically acceptable salts and the optical isomers of the products of formula (I), wherein:

A - for the preparation of a product or formula (I) in which Y denotes a CO radical and the other symbols are defined above, with the exception of the option in which Het denotes a 1,8-naphthyridin-2-yl radical substituted with a halogen atom at the 7-position, a product of general formula:

$CH_3$-CO-R (II)

in which R is defined as above, is reacted with a 3-hydroxyisoindolinone of general formula:

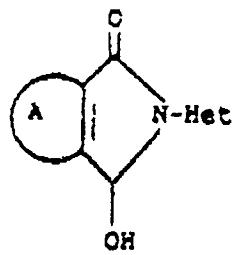

(III)

in which A and Het are defined as above, with the exception of the option in which Het denotes a 1,8-naphthyridin-2-yl radical substituted at the 7-position with a halogen atom, and the product obtained is then isolated and optionally converted according to known methods to another product of general formula (I) and, if so desired, where appropriate, to a pharmaceutically acceptable salt, or wherein:

B - for the preparation of a product of formula (I) in which Y denotes a CO radical and the other symbols are defined as above, with the exception of the option in which Het denotes a 1,8-naphthyridin-2-yl radical substituted at the 7-position with an alkyloxy or alkylthio radical, a $\beta$-keto ester of general formula:

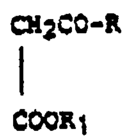

(IV)

in which R is defined as above and $R_1$ denotes an alkyl radical, is reacted with a product of general formula:

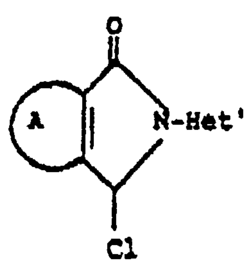

(V)

in which Het' has the definition given above for Het with the exception of denoting a 1,8-naphthyridin-2-yl radical substituted at the 7-position with an alkyloxy or alkylthio radical, and A is defined as above, a dealkyloxycarbonylation of the ester formed as an intermediate is then carried out, and the product obtained is isolated and optionally converted according to known methods to another product of general formula (I) and, if so desired where appropriate, to a pharmaceutically acceptable salt.

**Patentansprüche**

**Patentansprüche für folgende Vertragsstaaten : AT, BE, CH, DE, FR, GB, IT, LI, LU, NL, SE**

1. Neue Pyrrolderivate, dadurch gekennzeichnet, daß sie der allgemeinen Formel:

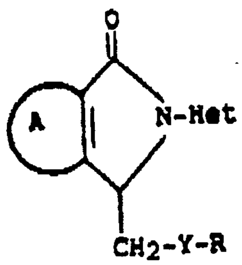

(I)

entsprechen, in welcher A zusammen mit dem Pyrrolring einen Isoindolinkern bildet, Het einen durch ein Halogenatom oder einen (1 bis 4 C) Alkyl-, (1 bis 4 C) Alkyloxy-, (1 bis 4 C) Alkylthiorest substituierten Naphthyridinylrest oder Quinolylrest, Y einen Rest CO, C = NOH oder CHOH darstellt und R einen 3 bis 10 Kohlenstoffatome in gerader oder verzweigter Kette enthaltenden Alkenylrest oder einen gegebenenfalls durch einen Alkyloxy-, Cycloalkyl- mit 3 bis 6 Kohlenstoffatomen, Dialkylamino-, Phenylrest substituierten Alkylrest darstellt oder R einen gegebenenfalls durch einen (1 bis 4 C) Alkylrest substituierten 4-Piperidylrest darstellt, wobei die Alkylreste selbstverständlich geradkettig oder verzweigt sind und ohne spezielle Angabe 1 bis 10 Kohlenstoffatome enthalten, sowie, falls es sie gibt, ihre pharmazeutisch zulässigen Salze und die optischen Isomeren der Verbindungen der Formel (I).

2. Verfahren zur Herstellung einer Verbindung nach Anspruch 1, in deren Formel Y einen CO-Rest darstellt und die übrigen Symbole die im Anspruch 1 angegebene Bedeutung haben mit Ausnahme eines in 7-Stellung durch ein Halogenatom substituierten 1,8-Naphthyridin-2-ylrestes für Het haben, dadurch gekennzeichnet, daß man eine Verbindung der allgemeinen Formel:

$CH_3$-CO-R (II)

in welcher R die im Anspruch 1 angegebene Bedeutung hat, mit einem 3-Hydroxy-isoindolinon der allgemeinen Formel;

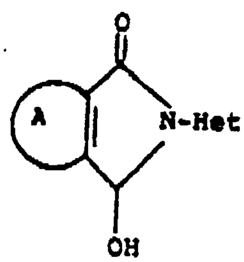

(III)

in welcher A und Het die im Anspruch 1 angegebene Bedeutung mit Ausnahme eines in 7-Stellung durch ein Halogenatom substituierten 1,8-Naphthyridin-2-ylrestes für Hat haben, umsetzt, dann die erhaltene Verbindung isoliert und die gegebenenfalls nach bekannten Methoden in eine andere Verbindung der allgemeinen Formel (I) und, gewünschtenfalls zutreffendenfalls in ein pharmazeutisch zulässiges Salz umwandelt.

3. Verfahren zur Herstellung einer Verbindung nach Anspruch 1, in deren Formel Y einen CO-Rest darstellt und die übrigen Symbole die im Anspruch 1 angegebene Bedeutung mit Ausnahme eines in 7-Stellung durch einer Alkyloxy- oder Alkylthiorest substituierten 1,8-Naphthyridin-2-ylrestes für Het haben, dadurch gekennzeichnet, daß man einen β-Ketoester der allgemeinen Formel

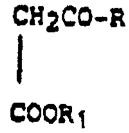

(IV)

in welcher R die im Anspruch 1 angegebene Bedeutung hat und $R_1$ einen Alkylrest darstellt, mit einer Verbindung der allgemeinen Formel:

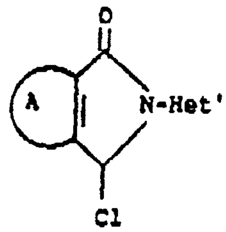

(V)

in welcher Het' die für Het im Anspruch 1 angegebene Bedeutung mit Ausnahme eines in 7-Stellung durch einen Alkyloxy- oder Alkylthiorest substituierten 1,8-Naphthyridin-2-ylrestes für Het hat und A die im Anspruch 1 angegebene Bedeutung hat, umsetzt, dann eine Desalkyloxycarbonylierung des als Zwischenprodukt gebildeten Esters durchführt, das erhaltene Produkt isoliert und es gegebenenfalls nach bekannten Methoden in ein anderes Produkt der allgemeinen Formel (I) und gewünschtenfalls zutreffendenfalls in ein pharmazeutisch zulässiges Salz umwandelt.

**4.** Pharmazeutische Präparate, dadurch gekennzeichnet, daß sie zumindest ein Produkt nach Anspruch 1 zusammen mit einem oder mehreren verträglichen und pharmazeutisch zulässigen Verdünnungsmitteln oder Zusatzstoffen enthalten.

**Patentansprüche für folgende Vertragsstaaten : ES, GR**

**1.** Verfahren zur Herstellung neuer Derivate der allgemeinen Formel:

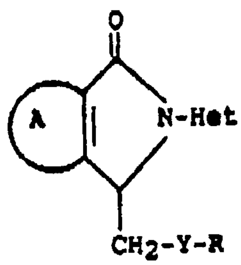

(I)

in welcher A zusammen mit dem Pyrrolring einen Isoindolinkern bildet, Het einen durch ein Halogenatom oder einen (1 bis 4 C) Alkyl-, (1 bis 4 C) Alkyloxy-, (1 bis 4 C) Alkylthiorest substituierten Naphthyridinylrest oder Quinolylrest, Y einen Rest CO, C=NOH oder CHOH darstellt und R einen 3 bis 10 Kohlenstoffatome in gerader oder verzweigter Kette enthaltenden Alkenylrest oder einen gegebenenfalls durch einen Alkyloxy-, Cycloalkyl- mit 3 bis 6 Kohlenstoffatomen, Dialkylamino-, Phenylrest substituierten Alkylrest darstellt oder R einen gegebenenfalls durch einen (1 bis 4 C) Alkylrest substituierten 4-Piperidylrest darstellt, wobei die Alkylreste selbstverständlich geradkettig oder verzweigt sind und ohne spezielle Angabe 1 bis 10 Kohlenstoffatome enthalten, sowie, falls es sie gibt, ihren pharmazeutisch zulässigen Salzen und den optischen Isomeren der Verbindungen der Formel (1), dadurch gekennzeichnet, daß man

A - Zur Herstellung einer Verbindung der Formel (I), in welcher Y einen CO-Rest darstellt und die übrigen Symbole die im) An-spruch 1 angegebene Bedeutung haben mit Ausnahme eines in 7-Stellung durch ein Halogenatom substituierten 1,8-Naphthyridin-2-ylrestes für Het haben, dadurch gekennzeichnet, daß man eine Verbindung der allgemeinen Formel:

$CH_3$-CO-R (II)

in welcher R die im Anspruch 1 angegebene Bedeutung hat, mit einem 3-Hydroxy-isoindolinon der allgemeinen Formel:

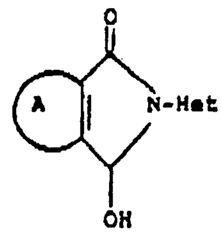

(III)

in welcher A und Het die im Anspruch 1 angegebene Bedeutung mit Ausnahme eines in 7-Stellung durch ein Halogenatom substituierten 1,8-Naphthyridin-2-ylrestes für Het haben, umsetzt, dann die erhaltene Verbindung isoliert und sie gegebenenfalls nach bekannten Methoden in eine andere Verbindung der allgemeinen Formel (1) und, gewünschtenfalls zutreffendenfalls in ein pharmazeutisch zulässiges Salz umwandelt, oder daß man

B - Zur Herstellung einer Verbindung der Formel (I), in welcher Y einen CO-Rest darstellt und die übrigen Symbole die im Anspruch 1 angegebene Bedeutung mit Ausnahme eines in 7-Stellung durch einen Alkyloxy- oder Alkylthiorest substituierten 1,8-Naphthyridin-2-ylrestes für Het haben, dadurch gekennzeichnet, daß man einen ß-Ketoester der allgemeinen Formel

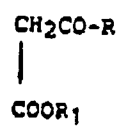

(IV)

in welcher R die im Anspruch 1 angegebene Bedeutung hat und $R_1$ einen Alkylrest darstellt, mit einer Verbindung der allgemeinen Formel:

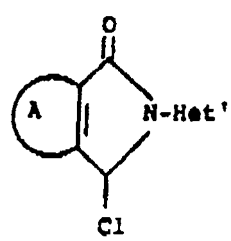

(V)

in welcher Het' die für Het im Anspruch 1 angegebene Bedeutung mit Ausnahme eines in 7-Stellung durch einen Alkyloxy- oder Alkylthiorest substituierten 1,8-Naphthyridin-2-ylrestes für Het hat und A die im Anspruch 1 angegebene Bedeutung hat, umsetzt, dann eine Desalkyloxycarbonylierung des als Zwischen-produkt gebildeten Esters durchführt, das erhaltene Produkt isoliert und es gegebenenfalls nach bekannten Methoden in ein anderes Produkt der allgemeinen Formel (I) und gewünschtenfalls zutreffendenfalls in ein pharmazeutisch zulässiges Salz umwandelt.